# EUROPÄISCHE PATENTANMELDUNG

(11) **EP 4 302 744 A1**
(43) Veröffentlichungstag der Anmeldung: **10.01.2024**
(21) Anmeldenummer: 23183093.6
(22) Anmeldetag: 03.07.2023
(51) Int. Cl.: A61K 8/02, A61F 13/15, A61K 8/11, A61K 8/35, A61K 8/37, A61K 8/92, A61K 8/9794, A61Q 19/00

(54) **HAUTAUFLAGE UND VERFAHREN ZUR HERSTELLUNG EINER HAUTAUFLAGE**

(30) Priorität: 05.07.2022 DE 102022116686
(71) Anmelder: SkinCura GmbH, 24568 Kaltenkirchen (DE)
(72) Erfinder: VOCK, Patrick, 22399 Hamburg (DE); VOCK, Kristina, 22399 Hamburg (DE)
(74) Vertreter: Lindner Blaumeier Patent- und Rechtsanwälte

(57) **Zusammenfassung**

Hautauflage, aufweisend einen flachen Grundkörper (2) aus einem synthetische Fasern (13) aufweisenden Textilmaterial, insbesondere Vlies (3), wobei an den hydrophilierten Fasern (13) bei mechanischer Einwirkung öffnende, hydrophile Mikrokapseln (14) angehaftet sind, die wenigstens einen medizinischen und/oder kosmetischen Wirkstoff (17, 18, 19) enthalten.

## Beschreibung

Die Erfindung betrifft eine Hautauflage, aufweisend einen flachen Grundkörper aus einem synthetische Fasern aufweisenden Textilmaterial, insbesondere Vlies. Daneben betrifft die Erfindung ein Verfahren zu Herstellung einer solchen Hautauflage.

Hautauflagen sind, beispielsweise als Wundauflage und/oder Kosmetikauflage im Stand der Technik grundsätzlich bekannt und weisen meist textile Grundkörper auf, die in direkten Kontakt mit der Haut eines Menschen zu bringen sind. Hierbei wird durch die Hautauflage insbesondere eine abdeckende Wirkung erzielt.

Zur tatsächlichen Behandlung von Hautbereichen, beispielsweise bei Reizungen und/oder leichten Entzündungen, insbesondere im Bereich der Epidermis, werden üblicherweise Salben und/oder Puder angewendet. Besonders problematisch erweisen sich diese Behandlungsvarianten im Bereich des Afters eines Menschen. Dies liegt insbesondere am Vorhandensein der Afterspalte, in der nicht nur ein Aufbringen von Wirksubstanzen, insbesondere Salben und/oder Pudern, äußerst kompliziert und unangenehm ist, sondern häufig auch die Salbe und/oder das Puder bei mechanischen Bewegungen wieder aus der Afterspalte, gegebenenfalls unter weiteren auftretenden Reizungen, herausgedrückt wird und/oder Kleidungsstücke verklebt.

Diese Problematik ist besonders relevant bei Kleinkindern und Babys, bei denen insbesondere beim Verwenden von Windeln Probleme wie Windeldermatitis und/oder sonstige Entzündungen und/oder Reizungen der Epidermis auftreten können. Zwar wurden im Stand der Technik bereits Windeleinlagen vorgeschlagen, die jedoch hauptsächlich dem Erhöhen der Saugkraft einer Windel, insbesondere einer Stoffwindel, dienen. Durch derartige Windeleinlagen, kann es ebenso zu den bereits genannten Reizungs- und/oder Entzündungsproblemen kommen.

Doch auch in anderen Hautbereichen, insbesondere solchen, die an anderen Hautbereichen anliegen können, ist das bislang bekannte Aufbringen von Salben und/oder Pudern mit ähnlichen Problemen behaftet. Insbesondere wird bei diesen Behandlungstechniken eine zeitlich punktuelle Aufbringung vorgenommen, sodass, insbesondere bei Verreiben und dergleichen, zu späteren Zeitpunkten eine deutlich reduzierte Wirkung vorliegen kann.

Der Erfindung liegt daher die Aufgabe zu Grunde, eine demgegenüber verbesserte, insbesondere in der Anwendung und hinsichtlich der Wirkstoffabgabe verbesserte, Hautauflage sowie eine Möglichkeit zu deren Herstellung anzugeben.

Zur Lösung dieser Aufgabe ist erfindungsgemäß eine Hautauflage, aufweisend einen flachen Grundkörper aus einem synthetische Fasern aufweisenden Textilmaterial, insbesondere aus einem Vlies, vorgesehen, die sich dadurch auszeichnet, dass an den hydrophilierten Fasern bei mechanischer Einwirkung öffnende, hydrophile Mikrokapseln angehaftet sind, die wenigstens einen medizinischen und/oder kosmetischen Wirkstoff enthalten.

Erfindungsgemäß wird mithin vorgeschlagen, eine Hautauflage, insbesondere eine Wundauflage und/oder eine Windeleinlage, zu schaffen, in welcher wenigstens ein Wirkstoff derart enthalten ist, dass er bei Benutzung der Hautauflage mit der Zeit freigegeben wird und entsprechend auf der Haut seine Wirkung, sei es medizinischer und/oder kosmetischer Art, entfalten kann. Hierzu wird konkret ein Grundkörper, insbesondere aus Vlies, vorgeschlagen, an dessen Fasern Mikrokapseln anhaften, was dadurch erreicht wird, dass die Fasern des Textilmaterials, insbesondere des Vlieses, hydrophiliert wurden. Als Hydrophilie wird im Allgemeinen die Fähigkeit einer Faser bezeichnet, Feuchtigkeit oder wasserverwandte Materialien aufzunehmen beziehungsweise eine anhaftende Bindung mit ihnen einzugehen. Durch den Prozess der Hydrophilierung synthetischer Fasern, wie er im Stand der Technik grundsätzlich bekannt ist, insbesondere im Hinblick auf hydrophile Farbstoffe, wird die Hydrophilie der Fasern hergestellt. Es entsteht eine Oberflächenmodifikation, die beispielsweise durch geeignete Textilveredlungsverfahren erreicht werden kann. Beispielsweise wurden auf Plasmatechnologie basierende Verfahren, Verfahren der radikalen Polymerisation und/oder die Verwendung von Makroinitiatoren als Primer vorgeschlagen. Derartige und andere Vorgehensweisen können auch im Rahmen der vorliegenden Erfindung eingesetzt werden, sodass die Fasern mithin eine Hydrophilie bereitstellende Oberflächenmodifikation aufweisen.

Diese Oberflächenmodifikation bietet dann die Grundlage für das Anhaften der Mikrokapseln, welche häufig auch bei nicht perfekter Kugelform als Mikrokügelchen bezeichnet werden. Die Mikrokapseln können beispielsweise eine Ausdehnung, insbesondere einen Durchmesser, von 10 - 500 µm, bevorzugt maximal 300 µm, aufweisen und definieren durch entsprechende Mikrokapselwände einen Innenraum, in dem der wenigstens eine Wirkstoff angeordnet ist. Dabei kann die Ausdehnung der Mikrokapseln, beispielsweise innerhalb des angegebenen Bereichs, variieren. Die Mikrokapseln sind derart ausgebildet, dass sie bei mechanischer Einwirkung, insbesondere durch die Haut, auf der die Hautauflage aufliegt, öffenbar sind und somit der wenigstens eine Wirkstoff an die Haut abgegeben werden kann. Hierbei sind die Mikrokapseln insbesondere derart ausgebildet, dass sie nicht bei bestimmter mechanischer Einwirkung allesamt unmittelbar öffnen, sondern dass ein zeitlicher, sukzessiver, statistischer Öffnungsprozess der Mikrokapseln stattfindet, mithin die Mikrokapseln über die Zeit ihren Wirkstoff an die Haut, auf der die Hautauflage aufliegt, abgeben können. Die Zahl der Mikrokapseln, ihre mechanischen Eigenschaften und die Menge an Wirkstoff, die in den Mikrokapseln enthalten ist, wird dabei so aufeinander abgestimmt, dass die gewünschte Behandlung durch den wenigstens einen Wirkstoff über einen gewünschten Zeitraum erreicht wird. Beispielsweise kann vorgesehen sein, dass 5 - 100 Mikrokapseln pro Quadratzentimeter der Grundkörperfläche vorgesehen sind. Entsprechend können beispielsweise bei einer Auflagefläche des Grundkörpers von 100 cm² 800 bis 2000 Mikrokapseln anhaften. Hinsichtlich der Wirkstoffmenge kann vorgesehen sein, 30 bis 80 g/l, beispielsweise 60 g/l, in einem Wirkstoffbad, durch das die hydrophilierten Fasern gezogen werden, vorzusehen.

Dabei sei noch angemerkt, dass nicht zwangsläufig die Wände der Mikrokapseln fest und formstabil sein müssen, sondern dass vielmehr bevorzugt ist, wenn die Bestandteile letztlich eine formveränderliche, beispielsweise viskose- und/oder gelartige und/oder elastische Hülle bilden. Auf diese Weise kann weitergehend eine, insbesondere zusätzliche, Reizung der Haut vermieden werden.

Der in den Mikrokapseln konservierte wenigstens eine Wirkstoff ist aufgrund der entsprechenden hydrophilen Eigenschaften mit dem Grundkörper vereint. Der wenigstens eine Wirkstoff kann über einen langen Zeitraum nach Applizierung an die betroffene Stelle der Haut abgegeben werden, sodass insbesondere auf problematische, herkömmliche Methoden der Behandlung verzichtet werden kann. Bei mechanischer Belastung, beispielsweise bei durch die Haut, auf der die Hautauflage aufliegt, ausgelöstem Knittern, Ziehen, Verformen und/oder Berühren des Grundkörpers mit den Mikrokapseln können sich diese öffnen, beispielsweise aufplatzen, und ihren Wirkstoff freisetzen. Auf diese Weise gewährleistet die Hautauflage auch, dass erst nach Applizierung der Hautauflage der Wirkstoff freigesetzt wird.

Mithin kann durch die erfindungsgemäße Hautauflage ein steigender Bedarf für kosmetische Behandlungslösungen, Wundheilungslösungen und/oder Pflegelösungen gedeckt werden, wobei Wirkstoffe im zu pflegenden und/oder zu behandelnden Bereich auch in schwierigen Situationen, wie beispielsweise bei Reisen, beim Sport oder Zuhause, einfach appliziert werden können. Auf die Verwendung von Salben, Cremes und/oder Pudern, die bis dato zur Behandlung und/oder Pflege eingesetzt wurden, kann somit verzichtet werden oder diese können ergänzend eingesetzt werden.

Wie bereits erwähnt, kann es sich bei der erfindungsgemäßen Hautauflage mit besonderem Vorteil um eine Windeleinlage handeln. Auf diese Weise ist eine flexibel einsetzbare Windeleinlage, die mit wenigstens einem geeigneten Wirkstoff kombiniert ist, bereitgestellt. Jedoch lässt sich die Hautauflage auch in anderen Einsatzgebieten vorteilhaft einsetzen, beispielsweise im Klinikbereich, zur Behandlung von Hautausschlägen und/oder Verbrennungen, im Kosmetikbereich, beispielsweise zur Applizierung und Pflege und/oder zur Behandlung sonstiger schwer zugänglicher Stellen, beispielsweise in Körperritzen. Weitere denkbare Einsatzbereiche umfassen auch die Pflege von gereizten Hautbereichen bei Tieren, das bedeutet, die Anwendung ist nicht auf Menschen beschränkt.

Zweckmäßigerweise kann das Vlies ein, insbesondere durch Erhitzen komprimiertes, Spinnvlies sein. Spinnvliese werden üblicherweise in Spinnverfahren hergestellt, bei welchen beispielsweise Kunststoffgranulat aufgeschmolzen werden kann und Fasern aerodynamisch oder mechanisch abgezogen werden können, wonach die Fadenschar zur Vlieslegung zugeführt wird, sodass ein Wirrvlies als Spinnvlies entsteht. Spinnvliese haben den Vorteil, identische physikalische Eigenschaften in allen Richtungen aufzuweisen und können in variablen Flächengewichten hergestellt werden. Insbesondere im Rahmen der vorliegenden Erfindung entsteht der Vorteil, dass die Dicke des Vlieses und die Faserdichte über das Vlies äußerst gleichförmig sind, was der Anwendung auf der Haut und als Träger für den wenigstens einen Wirkstoff zuträglich ist. Was die Fasern angeht, können diese, wie allgemein bekannt, auch im Rahmen der vorliegenden Erfindung aus Polyester und/oder Viskose und/oder Polypropylen bestehen. Bevorzugt können die Fasern aus reinem Polypropylen ohne Additive bestehen und/oder eine maximale Faserlänge von 70 mm und/oder eine Feinheit von maximal 35 µm (Micron) aufweisen.

Die speziellen hydrophilierten Fasern haben feuchtigkeitsregulierende, absorbierende Eigenschaften und sind hautfreundlich. Die Fasern der Auflage schützen die betreffende Stelle vor Austrocknung und schaffen durch die Fähigkeit zur Aufnahme von Exsudat ein ideales Milieu zur feuchten Wundheilung. Die Fasern stellen eine reibungsarme, hoch atmungsaktive Barriere gegen Bakterien und Viren dar. Zusätzlich werden Bakterien und Viren, die sich im betreffenden Bereich befinden, an der Hautauflage zum Teil gebunden und somit aus der Wunde Stück für Stück entfernt, da Bakterien oder Viren einen hydrophilen (=wasserliebenden) Anteil aufweisen und durch die hydrophilierten Fasern sich an die Fasern binden.

Die Hautauflage kann, je nach Anwendungsgebiet, beispielsweise eine rechteckige oder kreisförmige Form des flachen Grundkörpers aufweisen. Beispielsweise kann die Formgebung bekannten Wund- und Pflege-Pads nachempfunden sein. Mit besonderem Vorteil jedoch, insbesondere bei Ausbildung als Windeleinlage, kann die Hautauflage eine tropfenförmige, insbesondere zur Anwendung im Schritt eines Menschen geeignete, Form aufweisen. Eine Tropfenform vereinfacht die Applizierung in der Afterspalte, beispielsweise bei einem Kleinkind, sodass die gesamte Fläche der Afterspalte abgedeckt werden, jedoch aufgrund der Tropfenform im Bereich des Geschlechts zumindest im Wesentlichen keine Störung für den Träger bemerkbar ist, sodass ein hervorragender Tragekomfort gegeben ist.

Eine zweckmäßige Weiterbildung der Erfindung sieht vor, dass das Textilmaterial des Grundkörpers eine Grammatur von 25 - 40 g / m², insbesondere 30 g / m², aufweist. Versuche haben gezeigt, dass sich ein optimales Ergebnis hinsichtlich der Wirkstoffabgabe und des Tragekomforts bei einer Grammatur von 30 g / m² ergibt. Doch auch im sonstigen genannten Bereich ist eine hervorragende Wirkstoffabgabe und Tragequalität erreichbar. Der hier genannte Bereich hat sich auch als besonders vorteilhaft im Hinblick auf die Wirkung der hydrophilierten Fasern im Hinblick auf die Anziehung und Entfernung von Viren und Bakterien gezeigt, insbesondere im Zusammenspiel mit der Grammatur von 30 g / m².

Der wenigstens eine Wirkstoff kann einen entzündungs- und/oder reizungslindernden Wirkstoff und/oder hautpflegenden Wirkstoff umfassen. Wie bereits erwähnt, sind verschiedenste Anwendungsgebiete im Hinblick auf die Hautpflege und/oder die Wundbehandlung/Wundheilung im Rahmen der vorliegenden Erfindung denkbar, genau wie eine kosmetische Behandlung. Entsprechende, im Stand der Technik grundsätzlich bekannte Wirkstoffe, können auch im Rahmen der vorliegenden Erfindung verwendet werden.

So kann beispielsweise vorgesehen sein, dass der wenigstens eine Wirkstoff Mandelöl und/oder Aloe Vera und/oder Koenzym Q10 umfasst und/oder dass in den Mikrokapseln wenigstens ein Hilfsstoff, insbesondere Sojaöl, enthalten ist. Gerade bei Verwendung als Windeleinlage für die Behandlung von Reizungen und/oder Entzündungen der Haut und/oder die Hautpflege hat sich die Verwendung der Kombination Mandelöl, Aloe Vera und Koenzym Q10 als besonders nützlich erwiesen, wobei die Hauptwirkstoffkomponente das Mandelöl sein kann, während Aloe Vera und Koenzym Q10 in deutlich kleineren Mengen ausreichend sind. Als Hilfsstoff bietet sich hier insbesondere Sojaöl an. In Ausgestaltung ist es für die in den Mikrokapseln enthaltene Wirkstoffkombination beispielsweise denkbar, Mandelöl mit 40 - 60 Gew.-% und/oder Sojaöl mit 40-60 Gew.-% und/oder Aloe Vera mit 0,5-3 Gew.-% und/oder Koenzym Q10 mit 0,01 - 0,1 Gew.-% vorzusehen.

Mandelöl (auch Mandelkernöl beziehungsweise nach INCI: Prunus amygdalus dulcis (Sweet Almond) Oil) ist aus süßen Mandeln durch Kaltpressung gewonnenes fettes Pflanzenöl. Mandelöl ist sehr verträglich und vielseitig einsetzbar und für jeden Hauttyp geeignet. Dies gilt insbesondere, gerade bei einer Ausbildung der Hautauflage als Windeleinlage, auch für empfindliche, trockene Haut und Babyhaut. Mandelöl kann für eine weiche, geschmeidige und pflegende Anmutung sorgen, dringt gut in die Haut ein und durchfettet sie. Dies wird insbesondere bei trockener Haut als lindernd empfunden. Insgesamt kann gesagt werden, dass Mandelöl reizlindernd, feuchtigkeitsspendend und intensivpflegend wirkt.

Aloe Vera ist die allgemein gängige Bezeichnung für Erzeugnisse aus der Pflanzengattung Aloe Barbadensis, wobei im Rahmen der vorliegenden Erfindung insbesondere der Blätterextrakt (INCI: Aloe Barbadensis Leaf Extract) eingesetzt wird. Aloe Vera ist seit langer Zeit für seine vorteilhaften Gesundheits-, Schönheits- und Hautpflegeeigenschaften bekannt. Es hat heilende und antientzündliche Eigenschaften und kann sogar antiviral wirken.

Das Koenzym Q10, üblicherweise Ubiquinon-10 genannt, ist ein Quinon-Derivat mit lipophiler Isoprenoid-Seitenkette und ist strukturell verwandt mit Vitamin K und Vitamin E. Neben allgemeinen pflegenden Eigenschaften kann Q10 auch den Abbau von schädlichen Radikalen sicherstellen.

Wie erwähnt wurde, können auch Hilfsstoffe eingesetzt werden, beispielsweise als Wirkstoffträger. Hierbei bietet sich insbesondere die Verwendung von Sojaöl an. Sojaöl kann beispielsweise als Wirkstoffträger für lipidlösliche Substanzen und Pflanzeninhaltsstoffe verwendet werden. Nach INCI ist es als Glycine Soja Oil aufzuführen.

Als Bestandteil beziehungsweise Substanzen, aus denen die Mikrokapseln gebildet sind, kommen unterschiedliche Stoffe in Frage, die bevorzugt sowohl lipophile als auch hydrophile Eigenschaften aufweisen, um zum einem die Mikrokapseln um den wenigstens einen Wirkstoff (gegebenenfalls mit diesem tragenden Hilfsstoff) zu bilden, zum anderen aber auch die Anhaftung an die hydrophilierten Fasern zu erlauben. Denkbar sind auch Ansätze, in denen sich die Mikrokapseln aus wenigstens einer Substanz als Bestandteil zusammensetzen, die zur Zusammenlagerung durch Umschließung durch verschiedene physikalische und/oder chemische Prozesse tendiert. Dabei kann es, wie bereits erwähnt, besonders zweckmäßig sein, eine gewisse Elastizität und/oder Viskosität und/oder Gelartigkeit vorliegt, insbesondere derart, dass durch die mechanische Einwirkung ein Aufplatzen der Mikrokapseln eintreten kann, diese aber zugleich wenig reizend auf die Haut wirken.

In einer zweckmäßigen Ausgestaltung der vorliegenden Erfindung kann konkret vorgesehen sein, dass die Wand der Mikrokapseln ein mittelkettiges Triglycerid, insbesondere ein Caprylsäure-Triglycerid, und/oder Ethylacetat als Bestandteile aufweist. Caprylsäure-Triglycerid zeigt dabei die besten Ergebnisse bezüglich der Festigkeit und keine negativen Auswirkungen auf die Haut im Anwendungsbereich. Ethylacetat begünstigt vorteilhafterweise die Haltbarkeit und Festigkeit der Mikrokapseln. In einer besonders vorteilhaften Ausgestaltung können die Mikrokapseln also aus einer Mischung aus Caprylsäure-Triglycerid und Ethylacetat bestehen, beispielweise in einer Zusammensetzung von 90 - 99 % Caprylsäure-Triglycerid und 1 - 10 % Ethylacetat der Mikrokapselwand. Die Verwendung dieser beiden Substanzen führt beispielsweise zu blasenartigen und/oder folienartigen Mikrokapseln, die unter Einfluss der typischerweise bei Applikation, insbesondere im Gesäßbereich (beispielsweise als Windeleinlage), auftretenden mechanischen Einwirkungen mit einer bestimmten Wahrscheinlichkeit aufplatzen und den wenigstens einen Wirkstoff freigeben, sodass eine Wirkstofffreigabe über die Zeit umgesetzt werden kann.

In einer bevorzugten Weiterbildung kann vorgesehen sein, dass die Mikrokapselwand durch wenigstens ein Polymer oder eine Polymermischung verstärkt ist. Die zusätzliche Nutzung eines Polymers oder einer Polymermischung, welche beispielsweise nach einer ersten Ausbildung der Mikrokapseln im Herstellungsprozess zugegeben werden kann, erlaubt es letztlich, die Stärke der Mikrokapselwand bzw. deren Stabilität insbesondere in der Hinsicht einzustellen, über welchen Zeitraum die Öffnung der Mikrokapseln bei Benutzung der Hautauflage erfolgen soll. Würde in einem Beispiel die Öffnung ohne Polymerzusatz im Laufe von zwei bis sechs Stunden erfolgen, kann mittels des Polymerzusatzes dieser Zeitraum beispielhaft auf neun bis vierundzwanzig Stunden erweitert werden. Da Polymere selbst hydrophile Eigenschaften haben, verschlechtern sie die Anhaftung an die hydrophilierten Fasern nicht oder zumindest nicht merklich. Als besonders geeignet zur Erhöhung der Stärke der Mikrokapselwand hat sich eine Polymermischung aus 1,3-Propanediol, 2-ethyl-2-(hydroxymethyl)- (CAS 77-99-6) und 1,3-Diisocyanatomethylbenzene (CAS 26471-62-5) erwiesen, insbesondere im Verhältnis von 1:1. Der Anteil des Polymerzusatzes kann dabei gering gehalten werden, beispielsweise zwischen 0,1 Gew.-% und 5 Gew.-% der Gesamtmenge aus Wirkstoffen, ggf. einem Hilfsstoff, und Mikrokapseln betragen. Insbesondere bei der Herstellung, aber auch zu späteren Zeitpunkten, wirkt eine dünne Polymerschicht als Außenbestandteil der Mikrokapselwand auch schützend und/oder konservierend. Aufgrund von Herstellungstoleranzen wird ein breiter Bereich zwischen 0,1 % und 5 % angegeben, in dem die angegebenen Vorteile verlässlich erhalten werden.

In beispielhafter, konkreter Ausgestaltung der vorliegenden Erfindung kann vorgesehen sein, dass insgesamt die Mikrokapseln mit den Wirkstoffen und einem Hilfsstoff umfassen:
70 - 75 Gew.-% Caprylsäure-Triglycerid,
10 - 14 Gew.-% Mandelöl,
10 - 14 Gew.-% Sojaöl,
0,1 - 0,5 Gew.-% Aloe Vera, und
0,01 - 0,02 Gew.-% Koenzym Q10.

Bei der Verwendung von Polymerzusätzen zur Verstärkung der Mikrokapselwand kann hierzu beispielsweise noch 0,1 - 5 Gew.-% 1,3-Propanediol, 2-ethyl-2-(hydroxymethyl)- und 1,3-Diisocyanatomethylbenzene im Verhältnis 1:1 kommen.

Zusammensetzungen mit Mengen aus diesem Bereich haben sich als wirksam, angenehm und komfortabel, insbesondere für Windeleinlagen, erwiesen. Entsprechende Tests haben gezeigt, dass auch die bekannten Nutzungsvoraussetzungen erreichbar sind. Derartige Gesamtzusammensetzungen pflegen die Haut im Anwendungsbereich und wirken heilungsfördernd bei Reizungen, Rötungen und/oder leichten Entzündungen, wie sie beispielsweise bei Windeldermatitis auftreten können. Eine konkrete, als besonders vorteilhaft festgestellte Zusammensetzung kann vorsehen, dass die Mikrokapsel und die Wirkstoffmischung folgende Bestandteile, jeweils in Gew.-%, enthält: 73,6 % Caprylsäure Triglycerid, 12 % Mandelöl, 11,7 % Sojaöl, 2,14 % Ethylacetat, 0,31 % Aloe Vera - Blattextrakt und 0,01 % Coenzym Q10. Es sind jedoch auch andere, konkrete Zusammensetzungen vorteilhaft einsetzbar.

Zweckmäßigerweise kann vorgesehen sein, dass die Mikrokapseln mit dem wenigstens einen Wirkstoff beidseitig auf dem Grundkörper anhaften. Der Grundkörper kann also beidseitig mit den Mikrokapseln versehen werden, sodass keine klare Vorder- und Rückseite definiert werden muss. Zum anderen wurde festgestellt, dass auch auf der "Außenseite" bei Anwendung befindliche Mikrokapseln, genau wie mehr im Inneren anhaftende Mikrokapseln, ihren Wirkstoff durchaus zumindest teilweise durch das Textilmaterial, insbesondere Vlies, an die Haut weitergeben können.

Neben der Hautauflage betrifft die vorliegende Erfindung auch ein Verfahren zur Herstellung einer, insbesondere erfindungsgemäßen, Hautauflage, umfassend folgende Schritte:
- Bereitstellen eines flachen Grundkörpers aus einem synthetische Fasern aufweisenden Textilmaterial, insbesondere Vlies,
- Hydrophilierung der Fasern des Textilmaterials,
- Aufbringen eines Gemisches aus einer hydrophilen Flüssigkeit, insbesondere Wasser, und bei mechanischer Einwirkung öffnenden, hydrophilen Mikrokapseln, die wenigstens einen medizinischen und/oder kosmetischen Wirkstoff enthalten, auf den Grundkörper und Trocknen der Wundauflage, sodass die Mikrokapseln an den Fasern anhaften.

Sämtliche Ausführungen bezüglich der Hautauflage lassen sich analog auf das erfindungsgemäße Verfahren übertragen, mit welchem mithin ebenso die bereits genannten Vorteile erhalten werden können. Insbesondere gilt hinsichtlich der verwendbaren Substanzen und Anteile das bereits zur Hautauflage Ausgeführte entsprechend fort. So kann konkret beispielsweise vorgesehen sein, dass das Vlies in Spinnverfahren, mithin als Spinnvlies, hergestellt und/oder durch Erhitzung komprimiert wird. Nachdem bereits durch Verwendung eines Vlieses, insbesondere eines Spinnvlieses, das Ablösen von Fasern, die gegebenenfalls hautreizend wirken könnten, bereits äußerst weitgehend vermiesen wird, kann die Wahrscheinlichkeit hierfür durch eine Komprimierung mittels Erhitzung noch weiter reduziert werden und somit eine noch höhere Hautfreundlichkeit der Hautauflage bereitgestellt werden.

Zweckmäßigerweise kann nach der Hydrophilierung, vor dem Aufbringen des Gemischs mit den Mikrokapseln, der Grundkörper mit einer wasserabsorbierenden Substanz versehen werden, insbesondere mit einem wasserabsorbierenden Polymer. Nach dem Prozess der Hydrophilierung, wozu, wie bereits zur Hautauflage diskutiert, gängige Methoden des Standes der Technik eingesetzt werden können, kann mithin zur Erhöhung der Wasseraufnahmefähigkeit von glatten und dicht gewebten Vliesen aus synthetischen Fasern mit geringer Saugfähigkeit durch Auflagerung von wasserabsorbierenden Polymeren, besonders bevorzugt wasserabsorbierenden Polyamid-Emulsionen, eine weitere Verbesserung erreicht werden. Denn auf diese Weise breitet sich Feuchtigkeit leicht auf den Fasern aus und kann so insbesondere bei der Trocknung verdunsten, um den in den Mikrokapseln konservierten Wirkstoff fest mit dem Grundkörper zu vereinen. Neben der bevorzugt eingesetzten Polyamid-Emulsion kann auch ein sonstiges, superabsorbierendes Material (Superabsorber), eingesetzt werden und, insbesondere temporär, aufgebracht werden.

In einer besonders zweckmäßigen Weiterbildung des Verfahrens kann vorgesehen sein, dass zur Anfertigung des Gemisches zunächst eine erste Bestandteilgruppe umfassend wenigstens einen Teil des wenigstens einen Wirkstoffs in das Wasser dispergiert wird, wonach wenigstens eine zweite Bestandteilgruppe umfassend wenigstens einen die Mikrokapselwand bildenden Bestandteil zugegeben wird und, insbesondere durch Reaktion von wenigstens zwei Bestandteilen der Mikrokapselwand, Mikrokapseln um den wenigstens einen Wirkstoff ausgebildet werden. Mit derartigen Ansätzen, die bei Einbringung von Bestandteilen in Wasser, insbesondere bei Dispergieren, wenigstens teilweise ohnehin ausgelöst werden, ist eine besonders einfache Herstellung des Gemischs möglich. Hierzu seien zwei konkrete, denkbare Vorgehensweisen genauer erläutert.

In einer ersten, konkreten Vorgehensweise kann zunächst vorgesehen sein, nach der Bereitstellung von Wasser eine erste Substanz als Bestandteil der Mikrokapsel einzubringen, gemeinsam mit wenigstens einem des wenigstens einen Wirkstoffs und/oder wenigstens einem des wenigstens einen Hilfsstoffs, beispielsweise Sojaöl. Durch das Beigeben des wenigstens einen Wirkstoffs und/oder des wenigstens einen Hilfsstoffs bilden sich zunächst erste Wirkstoffkugeln, die im Folgenden die Mikrokapseln bilden werden und sich von dem Wasser absondern. In einem zweiten Schritt werden dann gegebenenfalls die weiteren Wirkstoffe und/oder weiteren Hilfsstoffe gemeinsam mit wenigstens einer weiteren Substanz als Bestandteil der Mikrokapsel beigegeben. Durch die Beigabe der Bestandteile der Mikrokapsel, insbesondere Caprylsäure-Triglycerid und/oder Ethylacetat gehen diese eine Verbindung mit den bereits enthaltenen Wirkstoffen ein. Die vorhandene Hydrophilie der Bestandteile der Mikrokapsel bevorzugt die, insbesondere mittels stattfindender Reaktion der Bestandteile der Mikrokapsel erfolgende, Ausbildung einer Mikrokapsel-Wand, die wie eine Membran bei entsprechender mechanischer Belastung, insbesondere durch die Haut bei Anwendung, den wenigstens einen Wirkstoff im Inneren freisetzen kann.

In einer anderen, bevorzugten Vorgehensweise kann zunächst wenigstens ein Teil des wenigstens einen Wirkstoffs eingebracht und dispergiert werden, sodass sich, wie beschrieben, einzelne Wirkstoffkugeln bilden. Hier kann auch bereits wenigstens ein Teil des wenigstens einen Hilfsstoffs eingebracht werden. Es können mehrere erste Bestandteilgruppen sukzessive eingebracht werden. In einem weiteren Schritt erfolgt dann die Zugabe aller Bestandteile der Mikrokapselwand,beispielsweise von Caprylsäure-Triglycerid und Ethylacetat, wobei in dieser zweiten Bestandteilgruppe dann auch gegebenenfalls noch verbleibende Teile des wenigstens einen Wirkstoffs und/oder des wenigstens einen Hilfsstoffs beigegeben werden können. Die Bestandteile selten sich, ggf. unter Reaktion von wenigstens zwei Bestandteilen, außen an die Wirkstoffkugeln an und bilden die Mikrokapselwand aus die wie eine Membran bei entsprechender mechanischer Belastung, insbesondere durch die Haut bei Anwendung, den wenigstens einen Wirkstoff im Inneren freisetzen kann. Dabei hat es sich bei Verwendung von Caprylsäure-Triglycerid als besonders vorteilhaft erwiesen, dieses zuletzt beizugeben.

In einer weiteren Variante kann zunächst, von reinem Wasser ausgehend, ein Hinzufügen des wenigstens einen Wirkstoffs, gegebenenfalls des wenigstens einen Hilfsstoffs und eines Teils der Bestandteile der Mikrokapselwand, insbesondere des Caprylsäure-Triglycerids und des Ethylacetats, erfolgen. Die gefertigte Lösung wird sodann für 20 bis 40 Minuten, beispielsweise 30 Minuten, stark durchmischt, beispielsweise mittels eines Rührwerks oder einer anderen Durchmischungseinrichtung. Hierbei bilden sich bereits die Mikrokapseln mit dem wenigstens einen Wirkstoff und gegebenenfalls dem wenigstens einen Hilfsstoff in deren Inneren. Die Mikrokapselwand bildet sich aus dem Caprylsäure-Triglycerid und dem Ethylacetat, wobei letzteres zudem konvervierend wirkt. In einem weiteren Unterschritt wird sodann ein Polymer oder eine Polymermischung, bevorzugt 1,3-Propanediol, 2-ethyl-2-(hydroxymethyl)- und 1,3-Diisocyanatomethylbenzene im Verhältnis 1:1, hinzugefügt, um die Mikrokapselwand zu verstärken. Hierzu wird erneut 150 bis 200, beispielsweise 180, Minuten stark, insbesondere Im Vergleich etwas schwächer, beispielsweise bei geringerer Drehzahl, durchmischt. Hierdurch verstärkt sich die Mikrokapselwand.

Das Aufbringen des Gemischs kann allgemein gesagt im Rahmen des Verfahrens durch ein Badverfahren und/oder ein Sprühverfahren erfolgen. Dabei wird bevorzugt der Grundkörper in das Gemisch eingetaucht, bis er vollkommen durch das Gemisch durchnässt ist. Danach kann er getrocknet werden, sodass das Wasser verdunstet und die Mikrokapseln an den Fasern anhaften.

Mit besonderem Vorteil findet die Trocknung bei 105 - 115 °C, insbesondere bei 110°C, statt, bevorzugt durch Infrarotwärme. Diese Temperatur hat sich als optimal erwiesen, um einen hinreichend schnellen Trocknungsprozess herbeizuführen, verhindert jedoch eine zu frühe Zerstörung der Mikrokapseln durch thermische Einwirkung. Diese kann, beispielsweise bei Verwendung von Caprylsäure-Triglycerid und Ethylacetat, bei höheren Temperaturen eintreten. Beispielsweise und allgemein kann im Rahmen der vorliegenden Erfindung nach einem Bad zum Aufbringen des Gemischs an und in den Grundkörper eine Trocknungsstrecke, beispielsweise eine Strecke von 15m, anschließen.

Weitere Vorteile und Einzelheiten der vorliegenden Erfindung ergeben sich aus den im folgenden beschriebenen Ausführungsbeispielen sowie anhand der Zeichnungen. Dabei zeigen:
- Figur 1: eine als Windeleinlage ausgebildete Hautauflage,
- Figur 2: eine schematische, vergrößerte Ansicht der Fasern in einem Vlies,
- Figur 3: einen schematischen Querschnitt durch eine Mikrokapsel,
- Figur 4: eine Zeichnung zur Applizierung der Windeleinlage, und
- Figur 5: einen Ablaufplan des erfindungsgemäßen Verfahrens.

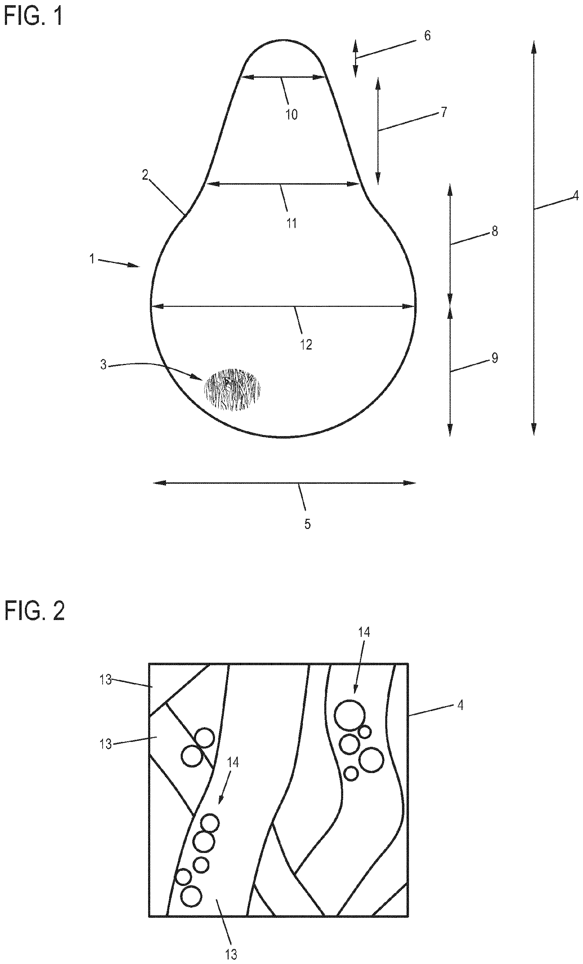

Figur 1 und Figur 2 erläutern ein Ausführungsbeispiel einer vorliegend als Windeleinlage 1 ausgebildeten Hautauflage. Die Windeleinlage 1 weist einen Grundkörper 2 aus Vlies 3 (der Übersichtlichkeit halber nur lokal gezeigt) auf, der vorliegend eine Tropfenform aufweist. Die Windeleinlage 1 ist vorliegend für Babys und Kleinkinder dimensioniert und weist eine Längsausdehnung 4 von 160 mm, eine Querausdehnung 5 von 109 mm sowie in Längsrichtung Teilausdehnungen 6 von 17 mm, 7 von 44 mm, 8 von 47 mm, 9 von 50mm und in Querrichtung Teilausdehnungen 10 von 35 mm, 11 von 68 mm und 12 von 109 mm auf.

Bei dem Vlies handelt es sich um ein Spinnvlies, wobei schematisch eine mikroskopische Vergrößerung der Fasern 13 mit anheftenden Mikrokapseln 14 in Figur 2 gezeigt ist. Die Anhaftung der Mikrokapseln 14, die hydrophil sind, an die Fasern 13 wird durch eine Hydrophilierung der Fasern 13 erreicht, also durch eine entsprechende Oberflächenstrukturierung derselben. Die Mikrokapseln können in ihrer Ausdehnung variieren und beispielsweise eine maximale Ausdehnung beziehungsweise einen Durchmesser von 10 - 500 µm aufweisen. Das Vlies 4 weist vorliegend eine Grammatur von 30 g / m² auf, wobei auch andere Grammaturen im Bereich von 25 - 40 g / m² denkbar sind.

Die Mikrokapseln 14 weisen eine Wand 15 auf, die vorliegend beispielhaft aus Caprylsäure-Triglycerid und Ethylacetat besteht. Die Wand 15 weist beispielsweise elastische Eigenschaften auf und kann bei mechanischer Einwirkung, wie sie beim Tragen der Windeleinlage 1 im Gesäßbereich auftreten können, öffnen, insbesondere Platzen, und somit eine in ihrem Innenraum angeordnete Wirkstoffmischung freigeben, sodass diese an der Haut wirken kann. Dabei sind die Substanzen, die Herstellung und die mechanischen Eigenschaften so gewählt, dass bei den zu erwarteten mechanischen Belastungen insgesamt die Wirkstoffmischung in gewünschten Dosen über einen Zeitraum freigegeben wird.

In weiteren Ausführungsbeispielen kann die Mikrokapselwand 15 ferner ein Polymer und eine Polymermischung, insbesondere als eine äußere Wand zur Verstärkung und somit Verlängerung des Zeitraums, über den die Wirkstoffmischung freigegeben wird, aufweisen, Die Polymermischung umfasst bevorzugt 1,3-Propanediol, 2-ethyl-2-(hydroxymethyl)- und 1,3-Diisocyanatomethylbenzene im Verhältnis 1:1.

Die Wirkstoffmischung umfasst vorliegend einen Hilfsstoff 16, der beispielsweise als Träger für die Wirkstoffe dienen kann. Der Hilfsstoff 16 kann beispielsweise Sojaöl sein. Als Wirkstoffe 17, 18 und 19 werden im vorliegenden Beispiel Mandelöl, Aloe Vera und Koenzym Q10 verwendet. Damit liegt vorliegend eine Gesamtzusammensetzung der Mikrokugeln 14 und der Wirkstoffmischung wie folgt vor, wobei die Angaben jeweils in Gew.-% sind: Caprylsäure-Triglycerid 73,6 %, Mandelöl 12 %, Sojaöl 11,7 %, Ethylacetat 2,14 %, Aloe Vera - Blattextrakt 0,31 % und Koenzym Q10 0,01 %. Eine derartige Zusammensetzung hat sich als besonders günstig erwiesen. Es sind jedoch auch andere Zusammensetzungen zur Pflege und/oder Behandlung der Haut bei Reizung und/oder leichten Entzündungen und/oder Rötungen denkbar, insbesondere im Rahmen der angegebenen Bereiche. Vorliegend sind bei der Herstellung 60 g Wirkstoffmischung auf einen Liter destilliertes Wasser zur Erzeugung eines Wirkstoffbades, durch das der Grundkörper 2 gezogen wurde, verwendet worden.

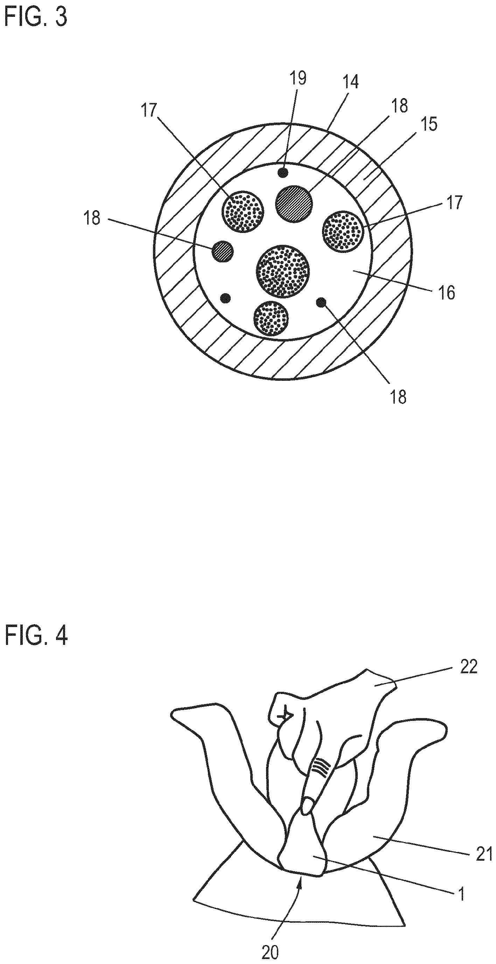

Figur 4 zeigt abstrahiert die Nutzung der Windeleinlage 1 im Gesäßbereich 20, konkret zumindest teilweise der Afterspalte, bei einem Baby 21, wobei durch eine Betreuungsperson (vgl. Hand 22) der tropfenförmige Grundkörper 2 im Gesäßbereich 20 des Babys 21 appliziert wird. Aufgrund der Tropfenform wird die gesamte Fläche der Afterspalte abgedeckt, wobei jedoch im Bereich des Geschlechts die Störung für den Träger minimiert wird und der Tragekomfort erhöht wird.

Neben den hier gezeigten Ausführungsbeispiel der Windeleinlage 1 sind selbstverständlich auch andere Arten von Hautauflagen erfindungsgemäß denkbar, beispielsweise Pflege- und/oder Wundpads und dergleichen, die bei unterschiedlichen Einsatzgebieten verwendet werden können, beispielsweise im klinischen Bereich (Behandlung von Hautausschlägen, Verbrennung, und dergleichen), im Kosmetikbereich (Applizierung von Kosmetika und/oder Pflege) beziehungsweise allgemein zur Behandlung schwer zugänglicher Stellen, beispielsweise in Körperritzen.

Dadurch, dass die Wirkstoffe 17, 18 und 19 in den Mikrokapseln 14 enthalten sind, die erst bei mechanischer Belastung über einen längeren Zeitraum hinweg schrittweise diese Wirkstoffe 17, 18, 19 freisetzen, ist eine hervorragende Zeitverteilung der Anwendung gegeben, wobei durchaus auch in Ausführungsbeispielen mehrere Anwendungen derselben Windeleinlage 1 beziehungsweise allgemein derselben Hautauflage denkbar sind.

Zur Hautfreundlichkeit und komfortablen Anwendung trägt weiter bei, dass eine Vliesvariante, hier ein Spinnvlies, verwendet wird, welches eine geschlossene Faserstruktur hat, um einen Faserverbleib im gereiztem, wunden, oder anderweitig behandelnden Bereich zu vermeiden. Hierzu kann beispielsweise auch eine Komprimierung durch Erhitzung erfolgt sein. Die hydrophilierten Fasern 13 selbst, die eine maximale Faserlänge von mindestens 70 mm und eine Feinheit von maximal 35 Micron aufweisen sowie aus einem reinen Polypropylen ohne Additive besetehen, haben feuchtigkeitsregulierende, absorbierende Eigenschaften und sind hautfreundlich. Die hydrophilierte Faserschicht der Windeleinlage 1 schützt den abgedeckten Anwendungsbereich vor Austrocknung und schafft durch die Aufnahme von Exsudat ein ideales Milieu zur feuchten Wundheilung. Insbesondere werden Bakterien und Viren, die sich im Anwendungsbereich befinden, an der Windeleinlage 1 zum Teil gebunden und somit aus der Wunde ferngehalten.

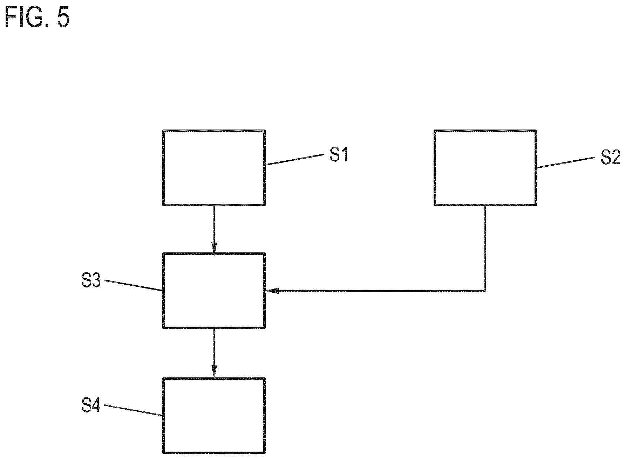

Die Herstellung solcher Hautauflagen wird nun im Hinblick auf Figur 5, die einen entsprechenden Ablaufplan zeigt, nochmals kurz erläutert.

Dabei wird in einem Schritt S1 zur Bereitstellung des Grundkörpers 2 das Textilmaterial, hier das Vlies, in einem Spinnverfahren, also als Spinnvlies, bereitgestellt. Danach kann das Vlies noch durch Erhitzen komprimiert werden. Als Fasern können Polyester und/oder Viskose und/oder Polypropylen verwendet werden. Es können übliche, grundsätzlich bekannte Spinnverfahren zur Herstellung von Spinnvliesen eingesetzt werden.

In einem auch parallel mit der Bereitstellung des Grundkörpers 2 durchführbaren Schritt S1 wird ein Gemisch vorbereitet, das vorliegend aus Wasser und den Mikrokapseln 14 besteht. Hierzu kann insbesondere eine mehrschrittige Hinzufügung von Bestandteilgruppen zu dem Wasser mit einer dazwischen liegenden Dispergierung erfolgen, um die Mikrokapselwände 15 zu bilden und das Wirkstoffgemisch zu umschließen. Ist das Gemisch so vorbereitet, wird in einem Schritt S3 der Grundkörper 2 in das Gemisch eingetaucht, insbesondere zur möglichst weitgehenden Durchnässung. Bevor dies geschieht, kann als ein optionaler Zwischenschritt der Grundkörper 2 auch mit einer wasserabsorbierenden Substanz versehen werden, beispielsweise mit einer wasserabsorbierenden Polyamid-Emulsion.

Der durchnässte Grundkörper 2 mit dem Wasser und den Mikrokapseln 14 wird dann in einem Schritt S4 einer Trocknungsstrecke zugeführt, wo das Wasser bei Temperaturen von 110 °C, die die Mikrokapseln 14 nicht beschädigen, verdunstet, sodass die Mikrokapseln 14 selbst an den Fasern 13 des Grundkörpers 2 anhaften.

Im Folgenden wird abschließend noch ein konkretes Beispiel dargelegt zur Herstellung von Mikrokapseln 14 in Wasser für 100.000 m² Textilmaterial mit einem Gewicht von 3.000 kg. Geht man von 60% Wasser aus und will man eine Wirkstoffkonzentration von 60 g/l erreichen, ergibt sich eine Menge an Nassbeschichtungsmaterial (Mikrokapseln 14 in Wasser) von 288 kg; zieht man eine Verflüchtigung von 20% in Betracht, ergeben sich 345,6 kg.

Um diese Menge herzustellen, werden zunächst 207,36 kg destilliertes Wasser bereitgestellt. In einem ersten Mischschritt werden in dieses Wasser
69,12 kg Caprylsäure-Triglycerid,
27,28 kg Mandelöl,
27,28 kg Sojaöl,
3,45 kg Ethylacetat,
3,45 kg Essigsäure,
1,3456 kg Aloe Vera, und
1,3456 kg Koenzym Q10
zugegeben. Die Essigsäure dient dabei der längeren Haltbarkeit der entstehenden Mischung, Es folgt eine starke Durchmischung bei 30 °C mittels eines Leitstrahlmischers bei 1450 Umdrehungen/Minute für wenigstens 30 Minuten.

In einem zweiten Mischschritt werden 4,968 kg einer Polymermischung aus 1,3-Propanediol, 2-ethyl-2-(hydroxymethyl)- und 1,3-Diisocyanatomethylbenzene im Verhältnis 1:1 zugegeben und das Gesamtgemisch wiederum bei 30°C mittels eines Leitstrahlmischers bei 950 Umdrehungen/Minute für wenigstens 180 Minuten stark durchmischt. So wird eine gleichmäßige Größenverteilung der Mikrokapseln 14 erreicht.

Der Gesamtzeitraum dieser Vorbereitung des Wassers mit den Mikrokapseln 14 darin beträgt wenigstens 3,5 Stunden.

Im Anschluss wird das Textilmaterial durch ein Tauchbecken mit obiger Mischung in einer Geschwindigkeit vom 30cm/min gezogen. Hierbei werden die hydrophilierten Fasern 13 im Zuführweg durch eine Flächenelektrode, die ein kontaktloses Aufladen mit ca. min. 1,5V gewährleistet, negativ elektrostatisch aufgeladen. Hierdurch wird eine bessere Anhaftung der hydrophilen Mikrokapseln 14 ermöglicht. Im Anschluss erfolgt eine Trocknung mit IR-Licht bei 110°C. Hierbei ist ein Abstand der Wärmequelle von max. 50cm zur Textilmaterialfläche zu gewährleisten.

Im Ergebnis erhält man ein Produkt mit folgenden Bestandtteilen von Mikrokapseln 14 und Wirkstoffmischung, jeweils in Gew.-%:
73,6 % Caprylsäure Triglycerid, 12 % Mandelöl, 11,7 % Sojaöl, 2,14 % Ethylacetat, 0,24 % Polymermischung, 0,31 % Aloe Vera - Blattextrakt und 0,01 % Coenzym Q10. Der geringere Anteil an Q10 im Endprodukt im Vergleich zu den Ausgangsstoffen beruht auf dessen stärkerer Verflüchtigung.

## Patentansprüche

1. Hautauflage, aufweisend einen flachen Grundkörper (2) aus einem synthetische Fasern (13) aufweisenden Textilmaterial, insbesondere Vlies (3), **dadurch gekennzeichnet, dass** an den hydrophilierten Fasern (13) bei mechanischer Einwirkung öffnende, hydrophile Mikrokapseln (14) angehaftet sind, die wenigstens einen medizinischen und/oder kosmetischen Wirkstoff (17, 18, 19) enthalten.

2. Hautauflage nach Anspruch 1, **dadurch gekennzeichnet, dass** das Vlies (3) ein, insbesondere durch Erhitzen komprimiertes, Spinnvlies ist und/oder die Fasern (13) aus Polyester und/oder Viskose und/oder Polypropylen bestehen.

3. Hautauflage nach Anspruch 1 oder 2, **dadurch gekennzeichnet, dass** die Hautauflage eine rechteckige oder kreisförmige oder tropfenförmige, insbesondere zur Anwendung im Schritt eines Menschen geeignete, Form aufweist und/oder eine Grammatur von 25 bis 40 g/m², insbesondere 30 g/m², aufweist.

4. Hautauflage nach einem der vorangehenden Ansprüche, **dadurch gekennzeichnet, dass** der wenigstens eine Wirkstoff (17, 18, 19) einen entzündungs- und/oder reizungslindernden Wirkstoff (17, 18, 19) und/oder einen hautpflegenden Wirkstoff (17, 18, 19) umfasst.

5. Hautauflage nach einem der vorangehenden Ansprüche, **dadurch gekennzeichnet, dass** der wenigstens eine Wirkstoff (17, 18, 19) Mandelöl und/oder Aloe Vera und/oder Koenzym Q10 umfasst und/oder in den Mikrokapseln (14) wenigstens ein Hilfsstoff, insbesondere Sojaöl, enthalten ist.

6. Hautauflage nach einem der vorangehenden Ansprüche, **dadurch gekennzeichnet, dass** die Wand (15) der Mikrokapseln (14) ein mittelkettiges Triglycerid, insbesondere ein Caprylsäuretriglycerid, und/oder Ethylacetat als Bestandteile aufweist, und/oder dass die Wand (15) der Mikrokapseln (14) durch wenigstens ein Polymer oder eine Polymermischung verstärkt ist.

7. Hautauflage nach einem der vorangehenden Ansprüche, **dadurch gekennzeichnet, dass** die Mikrokapseln (14) mit den Wirkstoffen (17, 18, 19) und einem Hilfsstoff umfassen:
70 bis 75 Gew.-% Caprylsäuretriglycerid,
10 bis 14 Gew.-% Mandelöl,
10 bis 14 Gew.-% Sojaöl,
0,1 bis 0,5 Gew.-% Aloe Vera, und
0,001 bis 0,02 Gew.-% Koenzym Q10.

8. Hautauflage nach einem der vorangehenden Ansprüche, **dadurch gekennzeichnet, dass** die Mikrokapseln (14) mit dem wenigstens einen Wirkstoff (17, 18, 19) beidseitig auf dem Grundkörper (2) anhaften.

9. Hautauflage nach einem der vorangehenden Ansprüche, **dadurch gekennzeichnet, dass** sie als Windeleinlage (1) ausgebildet ist.

10. Verfahren zur Herstellung einer Hautauflage, umfassend folgende Schritte:
- Bereitstellen eines flachen Grundkörpers (2) aus einem synthetische Fasern (13) aufweisenden Textilmaterial, insbesondere Vlies (3),
- Hydrophilierung der Fasern (13) des Textilmaterials,
- Aufbringen eines Gemisches aus einer hydrophilen Flüssigkeit, insbesondere Wasser, und bei mechanischer Einwirkung öffnenden, hydrophilen Mikrokapseln (14), die wenigstens einen medizinischen und/oder kosmetischen Wirkstoff (17, 18, 19) enthalten, auf den Grundkörper (2) und Trocknen der Wundauflage.

11. Verfahren nach Anspruch 10, **dadurch gekennzeichnet, dass** das Vlies (3) im Spinnverfahren hergestellt und/oder durch Erhitzung komprimiert wird.

12. Verfahren nach Anspruch 10 oder 11, **dadurch gekennzeichnet, dass** nach der Hydrophilierung, vor dem Aufbringen des Gemischs mit den Mikrokapseln (14), der Grundkörper (2) mit einer wasserabsorbierenden Substanz versehen wird, insbesondere mit einem wasserabsorbierenden Polymer.

13. Verfahren nach einem der Ansprüche 10 bis 12, **dadurch gekennzeichnet, dass** zur Anfertigung des Gemisches zunächst wenigstens eine erste Bestandteilgruppe umfassend wenigstens einen umfassend wenigstens einen Teil des wenigstens einen Wirkstoffs (17, 18, 19) in das Wasser dispergiert wird, wonach wenigstens eine zweite Bestandteilgruppe umfassend wenigstens einen die Mikrokapselwand bildenden Bestandteil zugegeben wird und, insbesondere durch Reaktion von wenigstens zwei Bestandteilen der Mikrokapselwand, die Mikrokapseln (14) um den wenigstens einen Wirkstoff (17, 18, 19) ausgebildet werden.

14. Verfahren nach einem der Ansprüche 10 bis 13, **dadurch gekennzeichnet, dass** das Aufbringen des Gemisches durch ein Badverfahren und/oder ein Sprühverfahren erfolgt.

15. Verfahren nach einem der Ansprüche 10 bis 14, **dadurch gekennzeichnet, dass** die Trocknung bei 105 bis 115 °C, insbesondere bei 110 °C, stattfindet.
